# EUROPEAN PATENT APPLICATION

(11) **EP 1 398 323 A1**
(43) Date of publication of application: **17.03.2004**
(21) Application number: 03016842.1
(22) Date of filing: 24.07.2003
(51) Int. Cl.: C07K 14/72

(54) **Murine androgen receptor complex-associated protein**

(30) Priority: 25.07.2002 US 206566
(71) Applicant: Taipei-Veterans General Hospital, Taipei (TW); TargetGen, Inc., Taipei 110 (TW)
(72) Inventor: Chang, Tai-Jay, Taipei (TW)
(74) Representative: Brandenburg, Thomas, Dr.

(57) **Abstract**

Androgen receptor complex-associated protein gene of the mouse and use thereof in the treatment of cancer.

SEQUENCE LISTING

<110> Chang, Tai-Jay

<120> TRANSGENIC ANIMALS EXPRESSING ANDROGEN
      RECEPTOR COMPLEX-ASSOCIATED PROTEIN

<130> 14321-003001

<140> US 10/206,566
<141> 2002-07-25

<150> US 09/781,693
<151> 2001-02-12

<150> US 60/262,312
<151> 2001-01-17

<160> 3

<170> FastSEQ for Windows Version 4.0

<210> 1
<211> 3340
<212> DNA
<213> Mus musculus

<220>
<221> CDS
<222> (218)...(2905)

<400> 1
gagagtatga ggcgagccgt ggcccgggtg cctctccgct cccggggaga aggcgcggcc      60
gtggctgccg ccctctgagt cgcggcgccg gcgaggcccc ggggcgcgcg catggtgctg     120
gtgccgctcg ggtgttgatc ggcctgtccc ctccctctct tcccctcccc accccccgcg     180
gtggtctccc ctttcccacc ccagcccttg cggagcc atg gct cgg agt ggc tcc      235
                                         Met Ala Arg Ser Gly Ser
                                          1               5

tgc ccg cac ctg ttg tgg gac gtg agg aaa agg tcc ctt ggg ctg gag       283
Cys Pro His Leu Leu Trp Asp Val Arg Lys Arg Ser Leu Gly Leu Glu
            10                  15                  20

gac ccg tcc cgg ctg agg agc cgc tac ctg gga aga aga gaa ttt atc       331
Asp Pro Ser Arg Leu Arg Ser Arg Tyr Leu Gly Arg Arg Glu Phe Ile
        25                  30                  35

caa aga tta aaa ctt gaa gca act tta aat gtg cat gat ggc tgt gtt       379
Gln Arg Leu Lys Leu Glu Ala Thr Leu Asn Val His Asp Gly Cys Val
    40                  45                  50

aat aca atc tgt tgg aat gac act gga gaa tat att tta tct ggc tct       427
Asn Thr Ile Cys Trp Asn Asp Thr Gly Glu Tyr Ile Leu Ser Gly Ser
 55                  60                  65                  70

gat gac act aaa ctt gta att agt aat cca tac agc aga aag gtt ttg       475
Asp Asp Thr Lys Leu Val Ile Ser Asn Pro Tyr Ser Arg Lys Val Leu
                 75                  80                  85

aca acc atc cgt tca ggg cat cga gca aat ata ttt agt gca aag ttt       523
Thr Thr Ile Arg Ser Gly His Arg Ala Asn Ile Phe Ser Ala Lys Phe
            90                  95                 100

ttg ccg tgc aca gat gat aag cag att gtg tct tgc tct gga gat gga       571
Leu Pro Cys Thr Asp Asp Lys Gln Ile Val Ser Cys Ser Gly Asp Gly

105                 110                 115

gtc ata ttt tat act aac att gag caa gat gca gaa act aac aga cag        619
Val Ile Phe Tyr Thr Asn Ile Glu Gln Asp Ala Glu Thr Asn Arg Gln
    120                 125                 130

tgc caa ttt acg tgc cat tat gga act act tat gag att atg act gta        667
Cys Gln Phe Thr Cys His Tyr Gly Thr Thr Tyr Glu Ile Met Thr Val
135                 140                 145                 150

cca aac gac cct tac acc ttt ctg tcc tgt ggt gaa gat gga act gtt        715
Pro Asn Asp Pro Tyr Thr Phe Leu Ser Cys Gly Glu Asp Gly Thr Val
                155                 160                 165

agg tgg ttt gac aca cgc atc aaa acc agt tgc aca aaa gaa gac tgt        763
Arg Trp Phe Asp Thr Arg Ile Lys Thr Ser Cys Thr Lys Glu Asp Cys
            170                 175                 180

aaa gat gat att tta atc aac tgt agg cgt gct gcc aca tct gtg gct        811
Lys Asp Asp Ile Leu Ile Asn Cys Arg Arg Ala Ala Thr Ser Val Ala
        185                 190                 195

att tgt ccc cca gta cca tat tac ctt gct gtg ggt tgt tct gac agc        859
Ile Cys Pro Pro Val Pro Tyr Tyr Leu Ala Val Gly Cys Ser Asp Ser
    200                 205                 210

tca gta cgg att tat gat cgg cga atg ctg ggc aca aga gct aca ggg        907
Ser Val Arg Ile Tyr Asp Arg Arg Met Leu Gly Thr Arg Ala Thr Gly
215                 220                 225                 230

aat tat gca ggc cga gga act act gga atg gtt gct cga ttt ata cct        955
Asn Tyr Ala Gly Arg Gly Thr Thr Gly Met Val Ala Arg Phe Ile Pro
                235                 240                 245

tct cat ctt agt aac aaa tca tgc aga gtg aca tca ctg tgt tac agt        1003
Ser His Leu Ser Asn Lys Ser Cys Arg Val Thr Ser Leu Cys Tyr Ser
            250                 255                 260

gaa gat ggt caa gag att ctt gtc agt tat tct tca gac tac atc tat        1051
Glu Asp Gly Gln Glu Ile Leu Val Ser Tyr Ser Ser Asp Tyr Ile Tyr
        265                 270                 275

ctt ttt gac ccc aaa gat gat act gca cga gaa ctt aaa act cct tct        1099
Leu Phe Asp Pro Lys Asp Asp Thr Ala Arg Glu Leu Lys Thr Pro Ser
    280                 285                 290

gca gag gag agg cga gaa gag tta cga cag cct cca gtt aag cgc ttg        1147
Ala Glu Glu Arg Arg Glu Glu Leu Arg Gln Pro Pro Val Lys Arg Leu
295                 300                 305                 310

aga ctt cgt ggt gat tgg tca gat act ggt ccc aga gca cgg cca gaa        1195
Arg Leu Arg Gly Asp Trp Ser Asp Thr Gly Pro Arg Ala Arg Pro Glu
                315                 320                 325

agt gaa cga gaa cga gat gga gag caa agt ccc aat gtg tca ctg atg        1243
Ser Glu Arg Glu Arg Asp Gly Glu Gln Ser Pro Asn Val Ser Leu Met
            330                 335                 340

cag aga atg tct gat atg tta tca agg tgg ttt gaa gaa gca agt gaa        1291
Gln Arg Met Ser Asp Met Leu Ser Arg Trp Phe Glu Glu Ala Ser Glu
        345                 350                 355

gtt gca caa agc aac aga gga aga gga aga cct cgg ccc aga ggt gga        1339
Val Ala Gln Ser Asn Arg Gly Arg Gly Arg Pro Arg Pro Arg Gly Gly
    360                 365                 370

aca aat cag cca gat gtt tca act ctt cct acg gtt cca tca agt cct        1387
Thr Asn Gln Pro Asp Val Ser Thr Leu Pro Thr Val Pro Ser Ser Pro

375                 380                 385                 390

aat ttg gaa gtg tgt gaa act gca atg gat gta gac atg cca gct gca      1435
Asn Leu Glu Val Cys Glu Thr Ala Met Asp Val Asp Met Pro Ala Ala
                395                 400                 405

ctt ctt cag cct tct aca tcc tct aca gat cca gtt cag gct cag gca      1483
Leu Leu Gln Pro Ser Thr Ser Ser Thr Asp Pro Val Gln Ala Gln Ala
            410                 415                 420

gcc aca gcc gcc ata gaa agc cct cgt tcc agc tcg ttg ctg tct tgc      1531
Ala Thr Ala Ala Ile Glu Ser Pro Arg Ser Ser Ser Leu Leu Ser Cys
        425                 430                 435

cca gac agt gaa ccg agg cag tct gtt gag gcg tct gga cac cat gca      1579
Pro Asp Ser Glu Pro Arg Gln Ser Val Glu Ala Ser Gly His His Ala
    440                 445                 450

cat cat cag tca gat tct cct tct tct gtg gtt aac aaa cag ctg gga      1627
His His Gln Ser Asp Ser Pro Ser Ser Val Val Asn Lys Gln Leu Gly
455                 460                 465                 470

tcc atg tca ctt gat gag caa cag gat aac agt aat gag agg ctg agc      1675
Ser Met Ser Leu Asp Glu Gln Gln Asp Asn Ser Asn Glu Arg Leu Ser
                475                 480                 485

ccc aaa cca ggg aca ggt gaa ccc gtt tta agt ttg cac tac agc aca      1723
Pro Lys Pro Gly Thr Gly Glu Pro Val Leu Ser Leu His Tyr Ser Thr
            490                 495                 500

gaa gga aca act aca agc aca ata aaa ctg aac ttt aca gat gaa tgg      1771
Glu Gly Thr Thr Thr Ser Thr Ile Lys Leu Asn Phe Thr Asp Glu Trp
        505                 510                 515

agc agt aca gcc tca agt tcc aga gga aat ggg agc cat tgc aaa tct      1819
Ser Ser Thr Ala Ser Ser Ser Arg Gly Asn Gly Ser His Cys Lys Ser
    520                 525                 530

gag ggt cag gaa gaa tgc ttg gtc cct ccg agc tct gtg cag cca ccg      1867
Glu Gly Gln Glu Glu Cys Leu Val Pro Pro Ser Ser Val Gln Pro Pro
535                 540                 545                 550

gaa gga gac agt gaa aca aga gct cct gaa gaa cta tca gag aaa gga      1915
Glu Gly Asp Ser Glu Thr Arg Ala Pro Glu Glu Leu Ser Glu Lys Gly
                555                 560                 565

aca ctt cca gaa aac ctc act caa aac cag ata gat aca gca caa ctt      1963
Thr Leu Pro Glu Asn Leu Thr Gln Asn Gln Ile Asp Thr Ala Gln Leu
            570                 575                 580

gat aac ttc cca gct gag cca ttg gat tct aac tca gga gag aag aat      2011
Asp Asn Phe Pro Ala Glu Pro Leu Asp Ser Asn Ser Gly Glu Lys Asn
        585                 590                 595

aac cca agt cag gac agc cct tgt ggg ctt cca gaa gaa ggc act ttg      2059
Asn Pro Ser Gln Asp Ser Pro Cys Gly Leu Pro Glu Glu Gly Thr Leu
    600                 605                 610

tct gaa aca gac agg gag act tgt gag cag gcc agc act gag agt gct      2107
Ser Glu Thr Asp Arg Glu Thr Cys Glu Gln Ala Ser Thr Glu Ser Ala
615                 620                 625                 630

acc agg cat gct agc acc aag cct gaa ctc cca tcc cag aca gaa gcc      2155
Thr Arg His Ala Ser Thr Lys Pro Glu Leu Pro Ser Gln Thr Glu Ala
                635                 640                 645

att gag cag gcc agc act gag agt gct acc agg cat acc agt gcc aat      2203
Ile Glu Gln Ala Ser Thr Glu Ser Ala Thr Arg His Thr Ser Ala Asn

            650                 655                 660

cct gaa ctc cca tcc cag aca gaa gcc ata gca cct tta gct cat gaa 2251
Pro Glu Leu Pro Ser Gln Thr Glu Ala Ile Ala Pro Leu Ala His Glu
        665                 670                 675

gac cca tct gcc agg gac tct gct ctc cag gac aca gat gac agc gat 2299
Asp Pro Ser Ala Arg Asp Ser Ala Leu Gln Asp Thr Asp Asp Ser Asp
    680                 685                 690

gat gat ccg gtc ttg atc cct ggt gca aga tac cga aca gga cct ggt 2347
Asp Asp Pro Val Leu Ile Pro Gly Ala Arg Tyr Arg Thr Gly Pro Gly
695                 700                 705                 710

gat aga cgc tcc gct gtt gcc cgc att cag gag ttc ttc agg agg aga 2395
Asp Arg Arg Ser Ala Val Ala Arg Ile Gln Glu Phe Phe Arg Arg Arg
                715                 720                 725

aaa gaa agg aaa gaa atg gaa gag ctg gat act ttg aac att agg agg 2443
Lys Glu Arg Lys Glu Met Glu Glu Leu Asp Thr Leu Asn Ile Arg Arg
            730                 735                 740

cca cta gta aag atg gtt tat aag ggc cac cgc aac tcc cgg aca atg 2491
Pro Leu Val Lys Met Val Tyr Lys Gly His Arg Asn Ser Arg Thr Met
        745                 750                 755

ata aaa gaa gcc aat ttc tgg ggt gct aac ttt gta atg agc ggt tcc 2539
Ile Lys Glu Ala Asn Phe Trp Gly Ala Asn Phe Val Met Ser Gly Ser
    760                 765                 770

gat tgt ggc cat atc ttc atc tgg gac cgg cac act gcg gag cat ttg 2587
Asp Cys Gly His Ile Phe Ile Trp Asp Arg His Thr Ala Glu His Leu
775                 780                 785                 790

atg ctt ctg gaa gct gat aat cat gtg gtc aac tgc ctg cag ccc cat 2635
Met Leu Leu Glu Ala Asp Asn His Val Val Asn Cys Leu Gln Pro His
                795                 800                 805

ccg ttt gac cca att cta gcc tca tct ggc ata gat tat gac ata aag 2683
Pro Phe Asp Pro Ile Leu Ala Ser Ser Gly Ile Asp Tyr Asp Ile Lys
            810                 815                 820

atc tgg tcg cca cta gaa gag tca aga att ttt aat cga aaa ctt gct 2731
Ile Trp Ser Pro Leu Glu Glu Ser Arg Ile Phe Asn Arg Lys Leu Ala
        825                 830                 835

gat gaa gtt ata act cgg aat gaa ctc acg ctg gaa gag act cgg aac 2779
Asp Glu Val Ile Thr Arg Asn Glu Leu Thr Leu Glu Glu Thr Arg Asn
    840                 845                 850

acc atc acc gtc cca gcc tct ttc atg ttg agg atg ttg gcg tca ctg 2827
Thr Ile Thr Val Pro Ala Ser Phe Met Leu Arg Met Leu Ala Ser Leu
855                 860                 865                 870

aat cat atc cga gct gac cgt ctg gag ggt gac aga tca gaa ggt tca 2875
Asn His Ile Arg Ala Asp Arg Leu Glu Gly Asp Arg Ser Glu Gly Ser
                875                 880                 885

ggt cag gag aat gaa aat gag gat gaa gaa taaagaactc cttggcaagc 2925
Gly Gln Glu Asn Glu Asn Glu Asp Glu Glu
            890                 895

acttagatgt tctgagattt gtatacgaca tttattatat ttttttcttt tacagaactt 2985
tagtgcaatt taaggctatg ggtttttttt tttctttttt tttggagttc ttccctattt 3045
tggggataac caaacattgg tttggaatga gtgtgtgcat gagttgggag agtgtgtaaa 3105
acaaagtaag caaaatgttt tttgaaacct tttgccgtgt atggagtccc aaaaaaaaaa 3165
aaaaaaaaaa aaaaaagcaa agtgcaatac ttcctgaccc tccgctgtgg gagcttggat 3225
caatgctgaa gtcattttca ttgtagtgaa aacgttggtt caaataaatt tctacacttg 3285

ccatttgcaa aaaaaaaaaa aaaaaaaaaa aaaaaaagcg gccgctgaat tctag 3340

<210> 2
<211> 2688
<212> DNA
<213> Mus musculus

<400> 2
atggctcgga gtggctcctg cccgcacctg ttgtgggacg tgaggaaaag gtcccttggg 60
ctggaggacc cgtcccggct gaggagccgc tacctgggaa gaagagaatt tatccaaaga 120
ttaaaacttg aagcaacttt aaatgtgcat gatggctgtg ttaatacaat ctgttggaat 180
gacactggag aatatatttt atctggctct gatgacacta aacttgtaat tagtaatcca 240
tacagcagaa aggttttgac aaccatccgt tcagggcatc gagcaaatat atttagtgca 300
aagtttttgc cgtgcacaga tgataagcag attgtgtctt gctctggaga tggagtcata 360
ttttatacta acattgagca agatgcagaa actaacagac agtgccaatt tacgtgccat 420
tatggaacta cttatgagat tatgactgta ccaaacgacc cttacacctt tctgtcctgt 480
ggtgaagatg gaactgttag gtggtttgac acacgcatca aaaccagttg cacaaaagaa 540
gactgtaaag atgatatttt aatcaactgt aggcgtgctg ccacatctgt ggctatttgt 600
cccccagtac catattacct tgctgtgggt tgttctgaca gctcagtacg gatttatgat 660
cggcgaatgc tgggcacaag agctacaggg aattatgcag gccgaggaac tactggaatg 720
gttgctcgat ttataccttc tcatcttagt aacaaatcat gcagagtgac atcactgtgt 780
tacagtgaag atggtcaaga gattcttgtc agttattctt cagactacat ctatcttttt 840
gaccccaaag atgatactgc acgagaactt aaaactcctt ctgcagagga gaggcgagaa 900
gagttacgac agcctccagt taagcgcttg agacttcgtg gtgattggtc agatactggt 960
cccagagcac ggccagaaag tgaacgagaa cgagatggag agcaaagtcc caatgtgtca 1020
ctgatgcaga gaatgtctga tatgttatca aggtggtttg aagaagcaag tgaagttgca 1080
caaagcaaca gaggaagagg aagacctcgg cccagaggtg gaacaaatca gccagatgtt 1140
tcaactcttc ctacggttcc atcaagtcct aatttggaag tgtgtgaaac tgcaatggat 1200
gtagacatgc cagctgcact tcttcagcct tctacatcct ctacagatcc agttcaggct 1260
caggcagcca cagccgccat agaaagccct cgttccagct cgttgctgtc ttgcccagac 1320
agtgaaccga ggcagtctgt tgaggcgtct ggacaccatg cacatcatca gtcagattct 1380
ccttcttctg tggttaacaa acagctggga tccatgtcac ttgatgagca acaggataac 1440
agtaatgaga ggctgagccc caaaccaggg acaggtgaac ccgttttaag tttgcactac 1500
agcacagaag gaacaactac aagcacaata aaactgaact ttacagatga atggagcagt 1560
acagcctcaa gttccagagg aaatgggagc cattgcaaat ctgagggtca ggaagaatgc 1620
ttggtccctc cgagctctgt gcagccaccg gaaggagaca gtgaaacaag agctcctgaa 1680
gaactatcag agaaaggaac acttccagaa aacctcactc aaaaccagat agatacagca 1740
caacttgata acttcccagc tgagccattg gattctaact caggagagaa gaataaccca 1800
agtcaggaca gcccttgtgg gcttccagaa gaaggcactt tgtctgaaac agacagggag 1860
acttgtgagc aggccagcac tgagagtgct accaggcatg ctagcaccaa gcctgaactc 1920
ccatcccaga cagaagccat tgagcaggcc agcactgaga gtgctaccag gcataccagt 1980
gccaatcctg aactcccatc ccagacagaa gccatagcac ctttagctca tgaagaccca 2040
tctgccaggg actctgctct ccaggacaca gatgacagcg atgatgatcc ggtcttgatc 2100
cctggtgcaa gataccgaac aggacctggt gatagacgct ccgctgttgc ccgcattcag 2160
gagttcttca ggaggagaaa agaaaggaaa gaaatggaag agctggatac tttgaacatt 2220
aggaggccac tagtaaagat ggtttataag ggccaccgca actcccggac aatgataaaa 2280
gaagccaatt tctggggtgc taactttgta atgagcggtt ccgattgtgg ccatatcttc 2340
atctgggacc ggcacactgc ggagcatttg atgcttctgg aagctgataa tcatgtggtc 2400
aactgcctgc agccccatcc gtttgaccca attctagcct catctggcat agattatgac 2460
ataaagatct ggtcgccact agaagagtca agaattttta atcgaaaact tgctgatgaa 2520
gttataactc ggaatgaact cacgctggaa gagactcgga acaccatcac cgtcccagcc 2580
tctttcatgt tgaggatgtt ggcgtcactg aatcatatcc gagctgaccg tctggagggt 2640
gacagatcag aaggttcagg tcaggagaat gaaaatgagg atgaagaa 2688

<210> 3
<211> 896
<212> PRT
<213> Mus musculus

<400> 3
Met Ala Arg Ser Gly Ser Cys Pro His Leu Leu Trp Asp Val Arg Lys
1               5                   10                  15
Arg Ser Leu Gly Leu Glu Asp Pro Ser Arg Leu Arg Ser Arg Tyr Leu
            20                  25                  30
Gly Arg Arg Glu Phe Ile Gln Arg Leu Lys Leu Glu Ala Thr Leu Asn
        35                  40                  45
Val His Asp Gly Cys Val Asn Thr Ile Cys Trp Asn Asp Thr Gly Glu
    50                  55                  60

Tyr Ile Leu Ser Gly Ser Asp Asp Thr Lys Leu Val Ile Ser Asn Pro
65                  70                  75                  80
Tyr Ser Arg Lys Val Leu Thr Thr Ile Arg Ser Gly His Arg Ala Asn
                85                  90                  95
Ile Phe Ser Ala Lys Phe Leu Pro Cys Thr Asp Asp Lys Gln Ile Val
            100                 105                 110
Ser Cys Ser Gly Asp Gly Val Ile Phe Tyr Thr Asn Ile Glu Gln Asp
        115                 120                 125
Ala Glu Thr Asn Arg Gln Cys Gln Phe Thr Cys His Tyr Gly Thr Thr
    130                 135                 140
Tyr Glu Ile Met Thr Val Pro Asn Asp Pro Tyr Thr Phe Leu Ser Cys
145                 150                 155                 160
Gly Glu Asp Gly Thr Val Arg Trp Phe Asp Thr Arg Ile Lys Thr Ser
                165                 170                 175
Cys Thr Lys Glu Asp Cys Lys Asp Asp Ile Leu Ile Asn Cys Arg Arg
            180                 185                 190
Ala Ala Thr Ser Val Ala Ile Cys Pro Pro Val Pro Tyr Tyr Leu Ala
        195                 200                 205
Val Gly Cys Ser Asp Ser Ser Val Arg Ile Tyr Asp Arg Arg Met Leu
    210                 215                 220
Gly Thr Arg Ala Thr Gly Asn Tyr Ala Gly Arg Gly Thr Thr Gly Met
225                 230                 235                 240
Val Ala Arg Phe Ile Pro Ser His Leu Ser Asn Lys Ser Cys Arg Val
                245                 250                 255
Thr Ser Leu Cys Tyr Ser Glu Asp Gly Gln Glu Ile Leu Val Ser Tyr
            260                 265                 270
Ser Ser Asp Tyr Ile Tyr Leu Phe Asp Pro Lys Asp Asp Thr Ala Arg
        275                 280                 285
Glu Leu Lys Thr Pro Ser Ala Glu Glu Arg Arg Glu Glu Leu Arg Gln
    290                 295                 300
Pro Pro Val Lys Arg Leu Arg Leu Arg Gly Asp Trp Ser Asp Thr Gly
305                 310                 315                 320
Pro Arg Ala Arg Pro Glu Ser Glu Arg Glu Arg Asp Gly Glu Gln Ser
                325                 330                 335
Pro Asn Val Ser Leu Met Gln Arg Met Ser Asp Met Leu Ser Arg Trp
            340                 345                 350
Phe Glu Glu Ala Ser Glu Val Ala Gln Ser Asn Arg Gly Arg Gly Arg
        355                 360                 365
Pro Arg Pro Arg Gly Gly Thr Asn Gln Pro Asp Val Ser Thr Leu Pro
    370                 375                 380
Thr Val Pro Ser Ser Pro Asn Leu Glu Val Cys Glu Thr Ala Met Asp
385                 390                 395                 400
Val Asp Met Pro Ala Ala Leu Leu Gln Pro Ser Thr Ser Ser Thr Asp
                405                 410                 415
Pro Val Gln Ala Gln Ala Ala Thr Ala Ala Ile Glu Ser Pro Arg Ser
            420                 425                 430
Ser Ser Leu Leu Ser Cys Pro Asp Ser Glu Pro Arg Gln Ser Val Glu
        435                 440                 445
Ala Ser Gly His His Ala His His Gln Ser Asp Ser Pro Ser Ser Val
    450                 455                 460
Val Asn Lys Gln Leu Gly Ser Met Ser Leu Asp Glu Gln Gln Asp Asn
465                 470                 475                 480
Ser Asn Glu Arg Leu Ser Pro Lys Pro Gly Thr Gly Glu Pro Val Leu
                485                 490                 495
Ser Leu His Tyr Ser Thr Glu Gly Thr Thr Thr Ser Thr Ile Lys Leu
            500                 505                 510
Asn Phe Thr Asp Glu Trp Ser Ser Thr Ala Ser Ser Ser Arg Gly Asn
        515                 520                 525
Gly Ser His Cys Lys Ser Glu Gly Gln Glu Glu Cys Leu Val Pro Pro
    530                 535                 540
Ser Ser Val Gln Pro Pro Glu Gly Asp Ser Glu Thr Arg Ala Pro Glu
545                 550                 555                 560
Glu Leu Ser Glu Lys Gly Thr Leu Pro Glu Asn Leu Thr Gln Asn Gln
                565                 570                 575
Ile Asp Thr Ala Gln Leu Asp Asn Phe Pro Ala Glu Pro Leu Asp Ser
            580                 585                 590
Asn Ser Gly Glu Lys Asn Asn Pro Ser Gln Asp Ser Pro Cys Gly Leu
        595                 600                 605

Pro Glu Glu Gly Thr Leu Ser Glu Thr Asp Arg Glu Thr Cys Glu Gln
    610                 615                 620
Ala Ser Thr Glu Ser Ala Thr Arg His Ala Ser Thr Lys Pro Glu Leu
625                 630                 635                 640
Pro Ser Gln Thr Glu Ala Ile Glu Gln Ala Ser Thr Glu Ser Ala Thr
                645                 650                 655
Arg His Thr Ser Ala Asn Pro Glu Leu Pro Ser Gln Thr Glu Ala Ile
            660                 665                 670
Ala Pro Leu Ala His Glu Asp Pro Ser Ala Arg Asp Ser Ala Leu Gln
        675                 680                 685
Asp Thr Asp Asp Ser Asp Asp Asp Pro Val Leu Ile Pro Gly Ala Arg
    690                 695                 700
Tyr Arg Thr Gly Pro Gly Asp Arg Arg Ser Ala Val Ala Arg Ile Gln
705                 710                 715                 720
Glu Phe Phe Arg Arg Arg Lys Glu Arg Lys Glu Met Glu Glu Leu Asp
                725                 730                 735
Thr Leu Asn Ile Arg Arg Pro Leu Val Lys Met Val Tyr Lys Gly His
            740                 745                 750
Arg Asn Ser Arg Thr Met Ile Lys Glu Ala Asn Phe Trp Gly Ala Asn
        755                 760                 765
Phe Val Met Ser Gly Ser Asp Cys Gly His Ile Phe Ile Trp Asp Arg
    770                 775                 780
His Thr Ala Glu His Leu Met Leu Leu Glu Ala Asp Asn His Val Val
785                 790                 795                 800
Asn Cys Leu Gln Pro His Pro Phe Asp Pro Ile Leu Ala Ser Ser Gly
                805                 810                 815
Ile Asp Tyr Asp Ile Lys Ile Trp Ser Pro Leu Glu Glu Ser Arg Ile
            820                 825                 830
Phe Asn Arg Lys Leu Ala Asp Glu Val Ile Thr Arg Asn Glu Leu Thr
        835                 840                 845
Leu Glu Glu Thr Arg Asn Thr Ile Thr Val Pro Ala Ser Phe Met Leu
    850                 855                 860
Arg Met Leu Ala Ser Leu Asn His Ile Arg Ala Asp Arg Leu Glu Gly
865                 870                 875                 880
Asp Arg Ser Glu Gly Ser Gly Gln Glu Asn Glu Asn Glu Asp Glu Glu
                885                 890                 895

## Description

### RELATED APPLICATIONS

This application is a continuation-in-part of and claims priority to U.S. Application Serial No. 09/781,693, filed February 12, 2001 and U.S. Provisional Application Serial No. 60/262,312, filed January 17, 2001, the contents of which are incorporated herein by reference.

### BACKGROUND

A variety of genes that are overexpressed in tumor cells relative to healthy cells have been identified. It is expected that the identification of such genes will provide drug targets for anti-cancer drug development and for cancer diagnostics. The number of steroid receptors (e.g., androgen receptors) in liver tumor cells appears to be increased relative to their adjacent healthy liver cells.

Steroid hormones generally exert their physiological effects by binding to their specific nuclear receptors to form complexes that in turn act as transcription factors. The complexes bind to specific nucleotide sequences (steroid responsive elements) in the promoters of steroid-responsive genes to facilitate transcription of those genes.

### SUMMARY

The invention is based on the discovery of a mouse gene encoding an androgen receptor complex-associated protein (ARCAP), which is 85% identical to human ARCAP. Human ARCAP has been found to be overexpressed in hepatoma cells (relative to adjacent normal cells), and binds to an androgen receptor and augments the ability of the receptor to transactivate an androgen-responsive gene. As a homolog to the human ARCAP gene, the mouse ARCAP gene can be used to generate a transgenic mouse that serves as an animal model for developing drugs to treat cancer (e.g., liver cancer).

The full-length mouse ARCAP cDNA (designated SEQ ID NO:1), with the start and stop codons underlined, is shown below: GAGAGTATGAGGCGAGCCGTGGCCCGGGTGCCTCTCCGCTCCCGGGGAGA 
AGGCGCGGCCGTGGCTGCCGCCCTCTGAGTCGCGGCGCCGGCGAGGCCCC
GGGGCGCGCGCATGGTGCTGGTGCCGCTCGGGTGTTGATCGGCCTGTCCC
CTCCCTCTCTTCCCCTCCCCACCCCCCGCGGTGGTCTCCCCTTTCCCACC
CCAGCCCTTGCGGAGCCATGGCTCGGAGTGGCTCCTGCCCGCACCTGTTG
TGGGACGTGAGGAAAAGGTCCCTTGGGCTGGAGGACCCGTCCCGGCTGAG
GAGCCGCTACCTGGGAAGAAGAATTTATCCAAAGATTAAAACTTGAAG
CAACTTTAAATGTGCATGATGGCTGTGTTAATACAATCTGTTGGAATGAC
ACTGGAGAATATATTTTATCTGGCTCTGATGACACTAAACTTGTAATTAG
TAATCCATACAGCAGAAAGGTTTTGACAACCATCCGTTCAGGGCATCGAG
CAAATATATTTAGTGCAAAGTTTTTGCCGTGCACAGATGATAAGCAGATT
GTGTCTTGCTCTGGAGATGGAGTCATATTTTATACTAACATTGAGCAAGA
TGCAGAAACTAACAGACAGTGCCAATTTACGTGCCATTATGGAACTACTT
ATGAGATTATGACTGTACCAAACGACCCTTACACCTTTCTGTCCTGTGGT
GAAGATGGAACTGTTAGGTGGTTTGACACACGCATCAAAACCAGTTGCAC
AAAAGAAGACTGTAAAGATGATATTTTAATCAACTGTAGGCGTGCTGCCA
CATCTGTGGCTATTTGTCCCCCAGTACCATATTACCTTGCTGTGGGTTGT
TCTGACAGCTCAGTACGGATTTATGATCGGCGAATGCTGGGCACAAGAGC
TACAGGGAATTATGCAGGCCGAGGAACTACTGGAATGGTTGCTCGATTTA
TACCTTCTCATCTTAGTAACAAATCATGCAGAGTGACATCACTGTGTTAC
AGTGAAGATGGTCAAGAGATTCTTGTCAGTTATTCTTCAGACTACATCTA
TCTTTTTGACCCCAAAGATGATACTGCACGAGAACTTAAAACTCCTTCTG
CAGAGGAGAGGCGAGAAGAGTTACGACAGCCTCCAGTTAAGCGCTTGAGA
CTTCGTGGTGATTGGTCAGATACTGGTCCCAGAGCACGGCCAGAAAGTGA
ACGAGAACGAGATGGAGAGCAAAGTCCCAATGTGTCACTGATGCAGAGAA
TGTCTGATATGTTATCAAGGTGGTTTGAAGAAGCAAGTGAAGTTGCACAA
AGCAACAGAGGAAGAGGAAGACCTCGGCCCAGAGGTGGAACAAATCAGCC
AGATGTTTCAACTCTTCCTACGGTTCCATCAAGTCCTAATTTGGAAGTGT
GTGAAACTGCAATGGATGTAGACATGCCAGCTGCACTTCTTCAGCCTTCT
ACATCCTCTACAGATCCAGTTCAGGCTCAGGCAGCCACAGCCGCCATAGA
AAGCCCTCGTTCCAGCTCGTTGCTGTCTTGCCCAGACAGTGAACCGAGGC
AGTCTGTTGAGGCGTCTGGACACCATGCACATCATCAGTCAGATTCTCCT
TCTTCTGTGGTTAACAAACAGCTGGGATCCATGTCACTTGATGAGCAACA
 
GGATAACAGTAATGAGAGGCTGAGCCCCAAACCAGGGACAGGTGAACCCG
TTTTAAGTTTGCACTACAGCACAGAAGGAACAACTACAAGCACAATAAAA
CTGAACTTTACAGATGAATGGAGCAGTACAGCCTCAAGTTCCAGAGGAAA
TGGGAGCCATTGCAAATCTGAGGGTCAGGAAGAATGCTTGGTCCCTCCGA
GCTCTGTGCAGCCACCGGAAGGAGACAGTGAAACAAGAGCTCCTGAAGAA
CTATCAGAGAAAGGAACACTTCCAGAAAACCTCACTCAAAACCAGATAGA
TACAGCACAACTTGATAACTTCCCAGCTGAGCCATTGGATTCTAACTCAG
GAGAGAAGAATAACCCAAGTCAGGACAGCCCTTGTGGCTTCCAGAAGAA
GGCACTTTGTCTGAAACAGACAGGGAGACTTGTGAGCAGGCCAGCACTGA
GAGTGCTACCAGGCATGCTAGCACCAAGCCTGAACTCCCATCCCAGACAG
AAGCCATTGAGCAGGCCAGCACTGAGAGTGCTACCAGGCATACCAGTGCC
AATCCTGAACTCCCATCCCAGACAGAAGCCATAGCACCTTTAGCTCATGA
AGACCCATCTGCCAGGGACTCTGCTCTCCAGGACACAGATGACAGCGATG
ATGATCCGGTCTTGATCCCTGGTGCAAGATACCGAACAGGACCTGGTGAT
AGACGCTCCGCTGTTGCCCGCATTCAGGAGTTCTTCAGGAGGAGAAAAGA
AAGGAAAGAAATGGAAGAGCTGGATACTTTGAACATTAGGAGGCCACTAG
TAAAGATGGTTTATAAGGGCCACCGCAACTCCCGGACAATGATAAAAGAA
GCCAATTTCTGGGGTGCTAACTTTGTAATGAGCGGTTCCGATTGTGGCCA
TATCTTCATCTGGGACCGGCACACTGCGGAGCATTTGATGCTTCTGGAAG
CTGATAATCATGTGGTCAACTGCCTGCAGCCCCATCCGTTTGACCCAATT
CTAGCCTCATCTGGCATAGATTATGACATAAAGATCTGGTCGCCACTAGA
AGAGTCAAGAATTTTTAATCGAAAACTTGCTGATGAAGTTATAACTCGGA
ATGAACTCACGCTGGAAGAGACTCGGAACACCATCACCGTCCCAGCCTCT
TTCATGTTGAGGATGTTGGCGTCACTGAATCATATCCGAGCTGACCGTCT
GGAGGGTGACAGATCAGAAGGTTCAGGTCAGGAGAATGAAAATGAGGATG
AAGAATAAAGAACTCCTTGGCAAGCACTTAGATGTTCTGAGATTTGTATA
CGACATTTATTATATTTTTTTCTTTACAGAACTTTAGTGCAATTTAAGG
CTATGGGTTTTTTTTTTCTTTTTTTTGGAGTTCTTCCCTATTTTGGGG
ATAACCAAACATTGGTTTGGAATGAGTGTGTGCATGAGTTGGGAGAGTGT
GTAAAACAAAGTAAGCAAAATGTTTTTGAAACCTTTTGCCGTGTATGGA
GTCCCAAAAAAAAAAAAAAAAAAAAAAAAAGCAAAGTGCAATACTTCCT
GACCCTCCGCTGTGGGAGCTTGGATCAATGCTGAAGTCATTTTCATTGTA
 
GTGAAAACGTTGGTTCAAATAAATTTCTACACTTGCCATTTGCAAAAAAA
AAAAAAAAAAAAAAAAAAAAAAGCGGCCGCTGAATTCTAG (SEQ ID NO:1)
 The nucleotide sequence encoding the mouse ARCAP protein (i.e., from the ATG start codon to the codon immediately before the stop codon in SEQ ID NO:1) is designated SEQ ID NO:2. The mouse ARCAP protein (designated SEQ ID NO:3) encoded by the above cDNA is shown below:

Met ala arg ser gly ser cys pro his leu leu trp asp val arg lys arg ser leu gly
leu glu asp pro ser arg leu arg ser arg tyr leu gly arg arg glu phe ile gln arg
leu lys leu glu ala thr leu asn val his asp gly cys val asn thr ile cys trp asn
asp thr gly glu tyr ile leu ser gly ser asp asp thr lys leu val ile ser asn pro
tyr ser arg lys val leu thr thr ile arg ser gly his arg ala asn ile phe ser ala
lys phe leu pro cys thr asp asp lys gln ile val ser cys ser gly asp gly val ile
phe tyr thr asn ile glu gln asp ala glu thr asn arg gln cys gln phe thr cys his
tyr gly thr thr tyr glu ile met thr val pro asn asp pro tyr thr phe leu ser cys
gly glu asp gly thr val arg trp phe asp thr arg ile lys thr ser cys thr lys glu
asp cys lys asp asp ile leu ile asn cys arg arg ala ala thr ser val ala ile cys
pro pro val pro tyr tyr leu ala val gly cys ser asp ser ser val arg ile tyr asp
arg arg met leu gly thr arg ala thr gly asn tyr ala gly arg gly thr thr gly met
val ala arg phe ile pro ser his leu ser asn lys ser cys arg val thr ser leu cys
tyr ser glu asp gly gln glu ile leu val ser tyr ser ser asp tyr ile tyr leu phe
asp pro lys asp asp thr ala arg glu leu lys thr pro ser ala glu glu arg arg glu
glu leu arg gln pro pro val lys arg leu arg leu arg gly asp trp ser asp thr gly
pro arg ala arg pro glu ser glu arg glu arg asp gly glu gln ser pro asn val ser
leu met gln arg met ser asp met leu ser arg trp phe glu glu ala ser glu val ala
gln ser asn arg gly arg gly arg pro arg pro arg gly gly thr asn gln pro asp val
ser thr leu pro thr val pro ser ser pro asn leu glu val cys glu thr ala met asp
val asp met pro ala ala leu leu gln pro ser thr ser ser thr asp pro val gln ala
gln ala ala thr ala ala ile glu ser pro arg ser ser ser leu leu ser cys pro asp
ser glu pro arg gln ser val glu ala ser gly his his ala his his gln ser asp ser
pro ser ser val val asn lys gln leu gly ser met ser leu asp glu gln gln asp asn
ser asn glu arg leu ser pro lys pro gly thr gly glu pro val leu ser leu his tyr
ser thr glu gly thr thr thr ser thr ile lys leu asn phe thr asp glu trp ser ser
thr ala ser ser ser arg gly asn gly ser his cys lys ser glu gly gln glu glu cys
leu val pro pro ser ser val gln pro pro glu gly asp ser glu thr arg ala pro glu
glu leu ser glu lys gly thr leu pro glu asn leu thr gln asn gln ile asp thr ala
gln leu asp asn phe pro ala glu pro leu asp ser asn ser gly glu lys asn asn pro
ser gln asp ser pro cys gly leu pro glu glu gly thr leu ser glu thr asp arg glu
thr cys glu gln ala ser thr glu ser ala thr arg his ala ser thr lys pro glu leu

pro ser gln thr glu ala ile glu gln ala ser thr glu ser ala thr arg his thr ser
ala asn pro glu leu pro ser gln thr glu ala ile ala pro leu ala his glu asp pro
ser ala arg asp ser ala leu gln asp thr asp asp ser asp asp pro val leu ile
pro gly ala arg tyr arg thr gly pro gly asp arg arg ser ala val ala arg ile gln
glu phe phe arg arg arg lys glu arg lys glu met glu glu leu asp thr leu asn ile
arg arg pro leu val lys met val tyr lys gly his arg asn ser arg thr met ile lys
glu ala asn phe trp gly ala asn phe val met ser gly ser asp cys gly his ile phe
ile trp asp arg his thr ala glu his leu met leu leu glu ala asp asn his val val
asn cys leu gln pro his pro phe asp pro ile leu ala ser ser gly ile asp tyr asp
ile lys ile trp ser pro leu glu glu ser arg ile phe asn arg lys leu ala asp glu
val ile thr arg asn glu leu thr leu glu glu thr arg asn thr ile thr val pro ala
ser phe met leu arg met leu ala ser leu asn his ile arg ala asp arg leu glu gly
asp arg ser glu gly ser gly gln glu asn glu asn glu asp glu glu (SEQ ID NO:3)

Accordingly, the invention features a substantially pure polypeptide or protein including an amino acid sequence at least 70% (e.g., at least 75, 80, 85, 90, 95, 98, or 100%) identical to SEQ ID NO:3. If the polypeptide includes a sequence that is 100% identical to SEQ ID NO:3, the polypeptide can contain up to 30 conservative amino acid substitutions. The invention also includes a substantially pure polypeptide encoded by a nucleic acid that hybridizes under stringent conditions to a probe the sequence of which is SEQ ID NO:2. The polypeptide binds to an androgen receptor and increases the ability of the androgen receptor to transactivate an androgen-responsive gene. These polypeptides can be used for producing ARCAP antibodies (either monoclonal or polyclonal). These antibodies in turn are useful for detecting the presence and distribution of ARCAP in tissues and in cellular compartments. For example, such antibodies can be used to diagnose cancerous liver tissue by determining whether ARCAP protein is expressed or overexpressed in the tissue. They can also be used to treat cancer (e.g., liver cancer) as described below.

The invention further features an isolated nucleic acid encoding a polypeptide of the invention, a vector including a nucleic acid of the invention, and a cell (in an animal model or in a culture) containing a nucleic acid of the invention. An example of a nucleic acid within the invention includes an isolated nucleic acid having a strand that hybridizes under stringent conditions to a single stranded probe, the sequence of which is SEQ ID NO:2 or the complement of SEQ ID NO:2. Such a nucleic acid can be at least 15 (e.g., at least 30, 50, 100, 200, 500, or 1000) nucleotides in length. These nucleic acids, vectors, and cells can be used for generating an animal model, diagnosing cancer (e.g., liver cancer), or producing the polypeptides of the invention. For instance, the nucleic acids of the invention can be used to diagnose liver cancer by determining whether ARCAP mRNA is being expressed or overexpressed in a tissue or cell. The nucleic acids can be used as primers in PCR-based detection methods, or as labeled probes in nucleic acid blots (e.g., Northern blots).

The invention also features a transgenic animal (e.g., a rodent such as a mouse) whose genome includes a transgene containing a nucleic acid of this invention and exhibits an increased androgen response as compared to a wild-type animal. The transgenic animal can be generated by introducing a nucleic acid of the invention into a cell such that a transcript is produced from the nucleic acid and the transcript is translated into a protein. These transgenic animals can be used as models for developing drugs to treat cancer (e.g., liver cancer).

In addition, the invention features a method of producing a polypeptide of the invention by culturing a cell described above, expressing the polypeptide in the cell, and isolating the polypeptide from the culture.

The invention further features a method of determining whether an animal sample (e.g., a blood sample) contains cancerous cells. The method involves providing a sample from an animal (e.g., a rodent such as a mouse), and determining the expression level of the ARCAP gene in the sample. If the expression level of the ARCAP gene in the sample is higher than that in a normal sample, it indicates that the animal sample contains cancerous cells (e.g., liver tumor cells). This method can be used for diagnosing cancer or monitoring treatment of cancer in an animal model.

Also within the scope of the invention is a method of treating cancer (e.g., liver cancer) in an animal (e.g., a rodent such as a mouse). The method involves identifying an animal that has a cancer cell expressing an ARCAP gene and treating the animal with a composition that blocks binding of ARCAP to an androgen receptor or decreases the ability of the androgen receptor to transactivate an androgen-responsive gene. The composition can contain an antibody of the invention or an anti-sense nucleic acid of the invention. This method can be used for screening drugs (including those used for gene therapy) and testing their efficacy in an animal model.

The term "substantially pure" as used herein in reference to a given polypeptide means that the polypeptide is substantially free from other biological macromolecules. The substantially pure polypeptide is at least 75% (e.g., at least 80, 85, 95, or 99%) pure by dry weight. Purity can be measured by any appropriate standard method, for example, by column chromatography, polyacrylamide gel electrophoresis, or HPLC analysis.

A "conservative amino acid substitution" is one in which an amino acid residue is replaced with another residue having a chemically similar side chain. Families of amino acid residues having similar side chains have been defined in the art. These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine), nonpolar side chains (e.g., alanine, valine, leucine, isoleucine, proline, phenylalanine, methionine, tryptophan), beta-branched side chains (e.g., threonine, valine, isoleucine) and aromatic side chains (e.g., tyrosine, phenylalanine, tryptophan, histidine).

By hybridization under "stringent conditions" is meant hybridization at 65°C, 0.5 X SSC, followed by washing at 45°C, 0.1 X SSC.

The "percent identity" of two amino acid sequences or of two nucleic acids is determined using the algorithm of Karlin and Altschul (Proc. Natl. Acad. Sci. USA 87:2264-2268, 1990), modified as in Karlin and Altschul (Proc. Natl. Acad. Sci. USA 90:5873-5877, 1993). Such an algorithm is incorporated into the NBLAST and XBLAST programs of Altschul et al. (J. Mol. Biol. 215:403-410, 1990). BLAST nucleotide searches are performed with the NBLAST program, score = 100, wordlength = 12. BLAST protein searches are performed with the XBLAST program, score = 50, wordlength = 3. Where gaps exist between two sequences, Gapped BLAST is utilized as described in Altschul et al. (Nucleic Acids Res. 25:3389-3402, 1997). When utilizing BLAST and Gapped BLAST programs, the default parameters of the respective programs (e.g., XBLAST and NBLAST) are used. See www.ncbi.nlm.nih.gov.

An "isolated nucleic acid" is a nucleic acid the structure of which is not identical to that of any naturally occurring nucleic acid or to that of any fragment of a naturally occurring genomic nucleic acid spanning more than three separate genes. The term therefore covers, for example, (a) a DNA which has the sequence of part of a naturally occurring genomic DNA molecule but is not flanked by both of the coding sequences that flank that part of the molecule in the genome of the organism in which it naturally occurs; (b) a nucleic acid incorporated into a vector or into the genomic DNA of a prokaryote or eukaryote in a manner such that the resulting molecule is not identical to any naturally occurring vector or genomic DNA; (c) a separate molecule such as a cDNA, a genomic fragment, a fragment produced by polymerase chain reaction (PCR), or a restriction fragment; and (d) a recombinant nucleotide sequence that is part of a hybrid gene, i.e., a gene encoding a fusion protein. Specifically excluded from this definition are nucleic acids present in mixtures of different (i) DNA molecules, (ii) transfected cells, or (iii) cell clones, e.g., as these occur in a DNA library such as a cDNA or genomic DNA library.

Other features or advantages of the present invention will be apparent from the following detailed description, and also from the claims.

### DETAILED DESCRIPTION

The invention relates to transgenic non-human animals that express the ARCAP gene and methods of using the animals for the development of drugs for treatment or prevention of liver cancer.

As used herein, "transgenic non-human animal" includes the founder transgenic non-human animals and progeny of the founders, as well as cells and tissues from such animals. Transgenic non-human animals can be farm animals such as pigs, goats, sheep, cows, horses, and rabbits, rodents such as rats, guinea pigs, and mice, and non-human primates such as baboons, monkeys, and chimpanzees. Transgenic pigs and mice are particularly useful.

A transgenic non-human animal of the invention contains a nucleic acid encoding an ARCAP protein integrated within its genome. As used herein, the term "ARCAP protein" refers to a wild-type ARCAP protein or a functionally equivalent variant, e.g., a fragment of the full-length protein.

It is contemplated that it may be useful to identify intron sequences, if any, in a genome and include them in a nucleic acid encoding an ARCAP protein.

In transgenic non-human animals of the invention, the nucleic acid is operably linked to a regulatory element that can promote expression of the ARCAP gene, e.g., in liver. As used herein, the term "operably linked" refers to the placement of the regulatory element and nucleic acid in such a manner that the nucleic acid is transcribed. The regulatory element can be a liver-specific promoter, such as a PEPCK and albumin promoter.

The invention also features expression vectors suitable for generating transgenic non-human animals of the invention. The expression vectors can include a promoter capable of mediating expression in liver operably linked to a nucleic acid encoding an ARCAP protein as described above.

Various techniques known in the art can be used to introduce expression vectors into non-human animals to produce the founder lines of the transgenic non-human animals. Such techniques include, but are not limited to, pronuclear microinjection (U.S Patent No. 4,873,191), retrovirus mediated gene transfer into germ lines (Van der Putten et al., Proc. Natl. Acad. Sci. USA, 82:6148, 1985), gene targeting into embryonic stem cells (Thompson et al., Cell, 56:313, 1989), electroporation of embryos (Lo, Mol. Cell. Biol., 3:1803, 1983), and transformation of somatic cells in vitro followed by nuclear transplantation (Wilmut et al., Nature, 385(6619):810-813, 1997; and Wakayama et al., Nature, 394:369-374, 1998). In one example, the expression vector is microinjected into an ovum or embryo of a non-human animal or into embryonic stem cells of a non-human animal.

Once transgenic non-human animals have been generated (e.g., a transgenic mouse generated according to the Current Protocol (Whey, USA) and Manuplate the Mouse Embryo (Hogan Beddington and Costantini Lacy, CSHL Press), expression of the ARCAP gene can be assessed using standard techniques. Initial screening can be accomplished by Southern blot analysis or PCR techniques to determine whether or not integration of the transgene has taken place. See, for example, sections 9.37-9.52 of Sambrook et al., 1989, "Molecular Cloning, A Laboratory Manual", second edition, Cold Spring Harbor Press, Plainview; NY, for a description of Southern analysis. Expression of the nucleic acid encoding an ARCAP protein in the tissues of the transgenic non-human animals can be assessed using techniques that include, but are not limited to, Northern blot analysis of tissue samples obtained from the animal, in situ hybridization analysis, and reverse-transcriptase PCR (RT-PCR).

Transgenic non-human animals of the invention can be used as models for liver cancer. In particular, these animals can be used to identify a compound or composition effective for treatment or prevention of liver cancer. Compounds or compositions can be identified by administering a test compound or composition to a transgenic non-human animal of the invention or by contacting the test compound or composition with an organ, a tissue (e.g., liver) or cells (e.g., liver cells) derived from the transgenic non-human animal. Effects of the test compound or composition on liver cancer of the transgenic non-human animal, organ, tissues or cells are evaluated. For example, the size of the cancer from clinical pathology identification can be assessed in the transgenic non-human animals. Test compounds or compositions that palliate the cancer symptoms can be effective for treatment or prevention of liver cancer.

Test compounds can be formulated into pharmaceutical compositions by admixing the compounds with pharmaceutically acceptable non-toxic excipients or carriers and administered to transgenic non-human animals of the invention by any route of administration. For example, parenteral routes such as subcutaneous, intramuscular, intravascular, intradermal, intranasal, inhalation, intrathecal, or intraperitoneal administration, and enteral routes such as sublingual, oral, or rectal administration can be used.

Without further elaboration, it is believed that one skilled in the art can, based on the description herein, utilize the present invention to its fullest extent. All publications recited herein are hereby incorporated by reference in their entirety.

### OTHER EMBODIMENTS

All of the features disclosed in this specification may be combined in any combination. Each feature disclosed in this specification may be replaced by an alternative feature serving the same, equivalent, or similar purpose. Thus, unless expressly stated otherwise, each feature disclosed is only an example of a generic series of equivalent or similar features.

From the above description, one skilled in the art can easily ascertain the essential characteristics of the present invention, and without departing from the spirit and scope thereof, can make various changes and modifications of the invention to adapt it to various usages and conditions. Thus, other embodiments are also within the scope of the following claims.

## Claims

1. A substantially pure polypeptide comprising an amino acid sequence at least 70% identical to SEQ ID NO:3, wherein the polypeptide binds to an androgen receptor and increases the ability of the androgen receptor to transactivate an androgen-responsive gene.

2. The polypeptide of claim 1, wherein the amino acid sequence is at least 80% identical to SEQ ID NO:3.

3. The polypeptide of claim 2, wherein the amino acid sequence is at least 90% identical to SEQ ID NO:3.

4. The polypeptide of claim 3, wherein the amino acid sequence is at least 95% identical to SEQ ID NO:3.

5. The polypeptide of claim 4, wherein the amino acid sequence is SEQ ID NO:3.

6. A substantially pure polypeptide comprising the amino acid sequence of SEQ ID NO:3, with up to 30 conservative amino acid substitutions, wherein the polypeptide binds to an androgen receptor and increases the ability of the androgen receptor to transactivate an androgen-responsive gene.

7. A substantially pure polypeptide encoded by a nucleic acid that hybridizes under stringent conditions to a probe, the sequence of which is SEQ ID NO:2, wherein the polypeptide binds to an androgen receptor and increases the ability of the androgen receptor to transactivate an androgen-responsive gene.

8. An isolated nucleic acid encoding a polypeptide of claim 1.

9. The nucleic acid of claim 8, wherein the amino acid sequence is SEQ ID NO: 3.

10. An isolated nucleic acid encoding a polypeptide of claim 6.

11. An isolated nucleic acid comprising a strand that hybridizes under stringent conditions to a single stranded probe, the sequence of which is SEQ ID NO:2 or the complement of SEQ ID NO:2.

12. The nucleic acid of claim 11, wherein the nucleic acid encodes a polypeptide that binds to an androgen receptor and increases the ability of the androgen receptor to transactivate an androgen-responsive gene.

13. The nucleic acid of claim 12, wherein the amino acid sequence of the polypeptide includes SEQ ID NO: 3.

14. The nucleic acid of claim 11, wherein the strand is at least 15 nucleotides in length.

15. A vector comprising a nucleic acid of claim 8.

16. The vector of claim 15, wherein the amino acid sequence is SEQ ID NO:3.

17. A vector comprising a nucleic acid of claim 10.

18. A vector comprising a nucleic acid of claim 11.

19. The vector of claim 18, wherein the nucleic acid encodes a polypeptide that binds to an androgen receptor and increases the ability of the androgen receptor to transactivate an androgen-responsive gene.

20. A cell comprising a nucleic acid of claim 8.

21. The cell of claim 20, wherein the amino acid sequence is SEQ ID NO:3.

22. A cell comprising a nucleic acid of claim 10.

23. A cell comprising a nucleic acid of claim 11.

24. The cell of claim 23, wherein the nucleic acid encodes a polypeptide that binds to an androgen receptor and increases the ability of the androgen receptor to transactivate an androgen-responsive gene.

25. A transgenic animal whose genome comprises a transgene containing a nucleic acid of claim 8, wherein the transgenic animal exhibits an increased androgen response as compared to a wild-type animal.

26. The transgenic animal of claim 25, wherein the animal is a rodent.

27. The transgenic animal of claim 26, wherein the animal is a mouse.

28. The transgenic animal of claim 25, wherein the amino acid sequence is SEQ ID NO:3.

29. The transgenic animal of claim 28, wherein the animal is a rodent.

30. The transgenic animal of claim 29, wherein the animal is a mouse.

31. A transgenic animal whose genome comprises a transgene containing a nucleic acid of claim 10, wherein the transgenic animal exhibits an increased androgen response as compared to a wild-type animal.

32. The transgenic animal of claim 31, wherein the animal is a rodent.

33. The transgenic animal of claim 32, wherein the animal is a mouse.

34. A transgenic animal whose genome comprises a transgene containing a nucleic acid of claim 11, wherein the transgenic animal exhibits an increased androgen response as compared to a wild-type animal.

35. The transgenic animal of claim 34, wherein the animal is a rodent.

36. The transgenic animal of claim 35, wherein the animal is a mouse.

37. A method of producing a polypeptide, the method comprising culturing a cell of claim 20, expressing the polypeptide in the cell, and isolating the polypeptide from the culture.

38. A method of producing a transcript, the method comprising introducing a nucleic acid of claim 11 into a cell, wherein a transcript is produced from the nucleic acid.

39. An isolated antibody that binds to a polypeptide comprising an amino acid sequence of SEQ ID NO:3.

40. A method of determining whether an animal sample contains cancerous cells, the method comprising:
providing a sample from an animal, and
determining an expression level of an androgen receptor complex-associated protein gene in the sample,
wherein the expression level of the androgen receptor complex-associated protein gene in the sample, if higher than that in a normal sample, indicates that the animal sample contains cancerous cells.

41. The method of claim 40, wherein the sample is a blood sample.

42. The method of claim 40, wherein the cancerous cells are liver tumor cells.

43. The method of claim 40, wherein the animal is a rodent.

44. The method of claim 43, wherein the animal is a mouse.

45. A method of treating cancer in an animal, the method comprising:
identifying an animal having a cancer cell that expresses an androgen receptor complex-associated protein gene, and
treating the animal with a composition that blocks binding of the androgen receptor complex-associated protein to an androgen receptor or decreases the ability of the androgen receptor to transactivate an androgen-responsive gene.

46. The method of claim 45, wherein the composition contains an antibody of claim 39.

47. The method of claim 45, wherein the composition contains a nucleic acid of claim 11.

48. The method of claim 45, wherein the cancer is a liver cancer.

49. The method of claim 45, wherein the animal is a rodent.

50. The method of claim 49, wherein the animal is a mouse.
